# EUROPEAN PATENT APPLICATION

(11) **EP 4 809 977 A1**
(43) Date of publication of application: **23.09.2026**
(21) Application number: 24891592.8
(22) Date of filing: 24.07.2024
(51) Int. Cl.: A61C 13/00, A61C 9/00, A61C 8/00, G06T 17/00, G06T 7/00, A61B 34/10, G06F 30/00, G06F 30/20

(54) **METHOD FOR MANUFACTURING PROSTHESIS FOR DENTAL IMPLANT USING DIGITAL LIBRARY**

(30) Priority: 17.11.2023 KR 20230159650
(71) Applicant: Innoden Co., Ltd., Siheung-si, Gyeonggi-do 15057 (KR)
(72) Inventor: JANG, Cheon Seok, Ansan-si Gyeonggi-do 15484 (KR)
(74) Representative: Cabinet Chaillot
(86) International application number: PCT/KR2024/010661
(87) International publication number: WO 2025/105643

(57) **Abstract**

The present invention relates to a method for manufacturing a prosthesis for a dental implant by using a digital library including ready-made abutment three-dimensional contour information regarding ready-made abutments having various specifications. According to an embodiment of the present invention, when designing a digital library including three-dimensional contour information of a ready-made abutment including a post having a cutting surface and/or a cutting groove formed on the outer peripheral surface thereof, the digital library including three-dimensional contour information of a ready-made abutment is designed by ignoring the cutting surface and/or the cutting groove such that, even if errors occur in the process of manufacturing the prosthesis in the dental laboratory, the occurrence of early contact on the periphery of the cutting surface and/or the cutting groove between the prosthesis and the post of the ready-made abutment can be prevented.

## Description

### [Technical Field]

The present invention relates to a method of manufacturing a crown of a dental implant, and more particularly, to a method of manufacturing a crown of a dental implant using a digital library including three-dimensional contour information of ready-made abutments for ready-made abutments having various specifications.

### [Background Art]

A dental implant system generally includes a fixture placed in an alveolar bone, an abutment that is fastened and fixed to the fixture and supports a pressure that corresponds to an occlusion force applied during mastication, and a crown (single/bridge) that restores an upper portion of the abutment, that is, a post, and restores aesthetic appearance similar to a natural tooth.

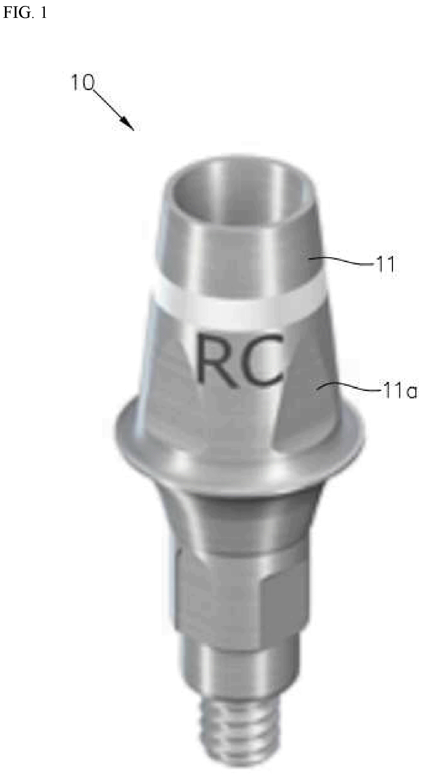

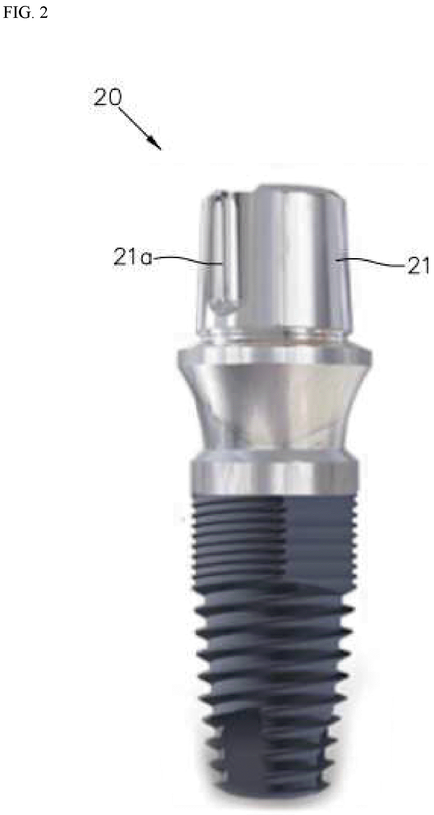

FIGS. 1 and 2 are views illustrating typical dental abutments, FIG. 1 illustrates a structure in which a cutting surface is formed on a post, and FIG. 2 illustrates a structure in which a cutting groove is formed on a post.

As in FIGS. 1 and 2, abutments 10 and 20 include posts 11 and 21 coupled (shape-matched) to an inner portion of a crown (not illustrated). One or a plurality of cutting surfaces 11a or cutting grooves 21a are formed on the posts 11 and 21 in the longitudinal direction (vertical direction) of the abutments 10 and 20. The cutting surfaces 11a or the cutting grooves 21a serve to prevent the crown from rotating about the posts 11 and 21 as axes in a state in which the posts 11 and 21 are coupled to the crown.

Methods of manufacturing a crown include an analog manufacturing method in which a crown is fabricated by taking an impression in an oral cavity using an impression material, forming a shape using wax, casting, and adding porcelain and a digital manufacturing method in which a crown is fabricated by scanning an oral cavity or a prefabricated tooth shape. Such methods correspond to one-time manufacturing methods. Unlike these, there is a method of manufacturing a crown using a dental digital library. The manufacturing method using the dental digital library has an advantage that it is more precise than the analog and digital manufacturing methods.

However, when a crown is manufactured using a dental digital library, various problems may be caused when the digital library is designed in a state in which a cutting surface or a cutting groove formed on a post of an abutment is reflected in the digital library. This may be a problem particularly when a bridge of crowns is manufactured, and for example, while this is not a problem when central axes of posts of abutments are formed to be parallel when manufacturing a bridge, when the central axes are not parallel, cutting surfaces or cutting grooves formed on the posts are also tilted instead of being parallel.

### [Related Art Documents]

### [Patent Documents]

(Patent Document 1) KR-1632378 B1, June 15, 2016
(Patent Document 2) KR 10-1726706 B1, April 07, 2017
(Patent Document 3) KR 10-1632373 B1, June 15, 2016
(Patent Document 4) KR 10-2385390 B1, April 06, 2022

### [Disclosure]

### [Technical Problem]

A post of an abutment generally has a tapered structure in which a diameter gradually decreases upward (see FIG. 1). Here, a cutting surface or a cutting groove formed on the post is tapered less than the post. For example, the cutting surface or the cutting groove is formed to be almost parallel to a central axis of the post (the central axis that vertically divides the post) in some cases. As a result, if an error occurs when a crown is manufactured in a dental laboratory process, the crown may not uniformly come into close contact with an outer surface of a post, and early contact in the form of point contact or line contact may occur between a specific portion of the crown and the post. That is, early contact such as point contact or line contact occurs between the crown and the abutment due to an error in the dental laboratory process.

Such early contact mostly occurs around a cutting surface or a cutting groove of a post when a crown is being coupled and fixed to an abutment. Here, when the degree to which a central axis of the post is tilted is negligible, the early contact issue may be addressed to some extent by stripping (removing) a portion of an inner surface of an accommodating portion of the crown, to which the post is shape-matched, during a dental laboratory process. However, when the degree to which the central axis of the post is tilted is high, because a majority of the inner surface has to be stripped to address the early contact issue, compatibility of the product crafted in the dental laboratory may be reduced. The crown manufactured in this way is not accurately coupled to the abutment. In particular, in an implant system in which a crown is coupled and fixed to an abutment using a screw type fixing member (a fixing screw) without using an adhesive, because it is not possible to strip the abutment, a problem in which it is substantially impossible to manufacture a bridge may occur.

Therefore, the present invention may be proposed to address a problem of early contact between a crown and an abutment that occurs in a conventional method of manufacturing a crown using a digital library.

One object of the present invention is to provide a method of manufacturing a crown of a dental implant using a digital library in which, when designing a digital library including three-dimensional contour information of an abutment to manufacture a crown, the digital library is designed to not reflect a cutting surface or a cutting groove formed on an outer periphery (an outer side surface) of a post so that the degree to which the outer periphery of the post of the abutment is tapered corresponds to the overall contour, and a crown of a dental implant is manufactured using the digital library designed in this way.

In addition, another object of the present invention is to provide a method of manufacturing a crown of a dental implant using a digital library in which a digital library is designed so that, in an implant system in which a cushioning member (an elastic body) is inserted between a crown and an abutment for the purpose of cushioning an occlusion force applied to the crown, an accommodating portion of the crown in which a post is accommodated is fabricated to have an accommodating space larger than the overall contour of the post by taking into consideration the thickness or volume of the cushioning member, and the cushioning material is stably inserted between the crown and the abutment.

Further, the objects of the present invention are not limited to those mentioned above, and various other objects may be additionally provided through embodiments described below and matters stated in the claims.

### [Technical Solution]

The present invention according to one embodiment for achieving the above objects provides a method of manufacturing a crown of a dental implant using a digital library, the method including: a process of designing a digital library including three-dimensional contour information of a ready-made abutment including a post having at least one of a cutting surface and a cutting groove formed on an outer periphery thereof; and a process of manufacturing a crown using the designed digital library, wherein, when designing the three-dimensional contour information of the ready-made abutment in the process of designing the digital library, the three-dimensional contour information of the ready-made abutment is designed so that, based on an intersection point at which an axial line of the post and a line perpendicular to the axial line of the post intersect, a distance between the intersection point and the outer periphery of the post is the same on the same plane along the circumference of the post.

In addition, the present invention according to another embodiment for achieving the above objects provides a method of manufacturing a crown of a dental implant using a digital library, the method including: a process of designing a digital library including three-dimensional contour information of a ready-made abutment including a post having at least one of a cutting surface and a cutting groove formed on an outer periphery thereof; and a process of manufacturing a crown using the designed digital library, wherein, when designing the three-dimensional contour information of the ready-made abutment in the process of designing the digital library, a three-dimensional contour of the post is designed in consideration of a thickness or a volume of a member interposed between the crown and the ready-made abutment.

In addition, the present invention according to still another embodiment for achieving the above objects provides a method of manufacturing a crown of a dental implant using a digital library, the method including: a process of designing a digital library including three-dimensional contour information of a ready-made abutment; and a process of manufacturing a crown using the designed digital library, wherein, when designing the three-dimensional contour information of the ready-made abutment in the process of designing the digital library, a three-dimensional contour of the post is designed in consideration of a thickness or a volume of a member interposed between the crown and the ready-made abutment.

In addition, when designing the three-dimensional contour information of the ready-made abutment in the process of designing the digital library, the three-dimensional contour of the post may be designed in a truncated cone shape.

In addition, when designing the three-dimensional contour information of the ready-made abutment in the process of designing the digital library, dental computer aided design (CAD) or dental computer aided manufacturing (CAM) may be used.

In addition, when designing the three-dimensional contour information of the ready-made abutment so that the distance between the intersection point and the outer periphery of the post is the same on the same plane along the circumference of the post in the process of designing the digital library, the three-dimensional contour information of the ready-made abutment may be designed so that a distance between the intersection point and a portion of the outer periphery of the post where the cutting surface or the cutting groove is formed matches a distance between the intersection point and a portion of the outer periphery of the post where the cutting surface or the cutting groove is not formed.

In addition, the post may be formed of a tapered structure in which a diameter gradually decreases upward.

In addition, the ready-made abutment may be formed of an integrated screw type structure or a detachable screw type structure.

Further, the crown may be a single crown or a bridge crown.

### [Advantageous Effects]

As described above, according to a method of manufacturing a crown of a dental implant using a digital library according to an embodiment of the present invention, when designing a digital library including three-dimensional contour information of a ready-made abutment including a post having a cutting surface and/or a cutting groove formed on an outer periphery thereof, the digital library including the three-dimensional contour information of the abutments is designed to ignore the cutting surface and/or the cutting groove, thereby preventing the occurrence of early contact between a crown and the post of the abutment around the cutting surface and/or the cutting groove even when an error occurs in the process of manufacturing the crown in a dental laboratory.

In addition, according to a method of manufacturing a crown of a dental implant using a digital library according to an embodiment of the present invention, a digital library is designed so that, in an implant system in which a cushioning member (an elastic body) is inserted between a crown and an abutment for the purpose of cushioning an occlusion force applied to the crown, an accommodating portion of the crown is fabricated to have an accommodating space larger than a contour of a post by taking into consideration the thickness or volume of the cushioning member, thereby allowing the cushioning material to be stably inserted between the crown and the abutment.

### [Description of Drawings]

FIG. 1 is a view illustrating a structure in which a cutting surface is formed on a post of a ready-made abutment.
FIG. 2 is a view illustrating a structure in which a cutting groove is formed on a post of a ready-made abutment.
FIG. 3 is a flowchart illustrating a method of manufacturing a crown of a dental implant according to an embodiment of the present invention.
FIG. 4 is a view illustrating one example of a ready-made abutment.
FIG. 5 is a cross-sectional view along direction "A-A" shown in FIG. 4.
FIG. 6 is a top view of the abutment illustrated in FIG. 4.
FIG. 7 is a view illustrating a cross-section of a crown according to one example of the present invention.
FIG. 8 is a view illustrating an implant system including a crown manufactured through an embodiment of the present invention.
FIG. 9 is a view for describing a method of designing a digital library according to another example of the present invention.
FIG. 10 is a cross-sectional view along direction "B-B" of an implant system including a crown manufactured through the method of FIG. 9.

### [Modes of the Invention]

Hereinafter, advantages and features of the present invention and methods of achieving them will become apparent from the following description of embodiments with reference to the accompanying drawings. In addition, the same reference numerals refer to the same components throughout the specification. In addition, the size and shape of each component illustrated in each drawing may be exaggerated for convenience of description, and it is not intended to limit the present invention. Further, the phrase "A and/or B" may mean both A and B or any one of A and B.

A dental digital library may be a database including three-dimensional contour information of ready-made abutments obtained from actual ready-made abutments fabricated in various specifications. Here, the ready-made abutments are actual abutments fabricated by different manufacturers, and the three-dimensional contour information of the ready-made abutments is three-dimensional vector data that allows the contour and structure of the actually fabricated ready-made abutments to be identically reproduced. That is, the digital library provides three-dimensional contour information of a ready-made abutment that serves as a reference when designing a crown. In an embodiment of the present invention, the three-dimensional contour information is obtained using a design method using dental computer aided design (CAD) or dental computer aided manufacturing (CAM) instead of a design method using a method in which an actually fabricated ready-made abutment is directly scanned. In addition, three-dimensional contour information of a ready-made abutment may be stored in a digital library while an independent item number is assigned thereto according to its specifications. For example, the same item number may be assigned to several abutments having the same specifications, and different item numbers may be assigned to several abutments having different specifications.

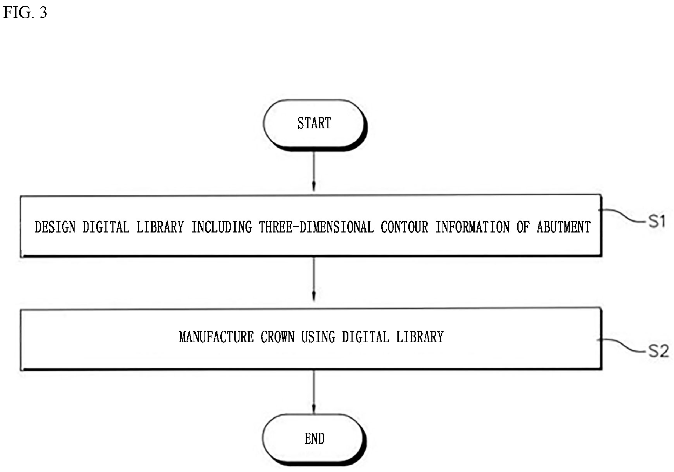

FIG. 3 is a flowchart illustrating a method of manufacturing a crown of a dental implant according to an embodiment of the present invention.

Referring to FIG. 3, a method of manufacturing a crown of a dental implant according to an embodiment of the present invention includes a process S1 of designing a digital library including three-dimensional contour information of a ready-made abutment to which a crown to be manufactured will be coupled and fixed and a process S2 of manufacturing the crown using the three-dimensional contour information of the ready-made abutment that is included in the designed digital library.

A digital library including three-dimensional contour information of a ready-made abutment may be designed using CAD or CAM by a manufacturer that produces and manufactures ready-made abutments. The designed digital library is provided to a dental laboratory, and in the dental laboratory, a crown is manufactured based on three-dimensional contour information of an abutment that is included in the designed digital library.

When the digital library is designed using CAD or CAM, a three-dimensional contour of a ready-made abutment is designed in a state in which the shape of a cutting surface and/or a cutting groove formed on a post of the ready-made abutment is excluded so that information of the cutting surface and/or the cutting groove formed on the post of the ready-made abutment is not reflected in three-dimensional contour information of the ready-made abutment. That is, even when a cutting surface and/or a cutting groove are/is formed on a post of an actual ready-made abutment, three-dimensional contour information of the ready-made abutment is obtained by designing the entire shape of the post while ignoring the cutting surface and/or the cutting groove when designing the three-dimensional contour of the actual ready-made abutment.

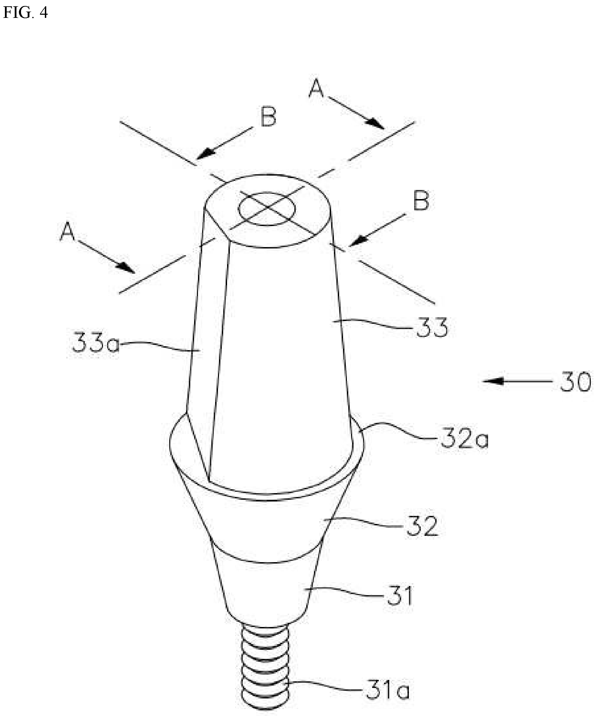

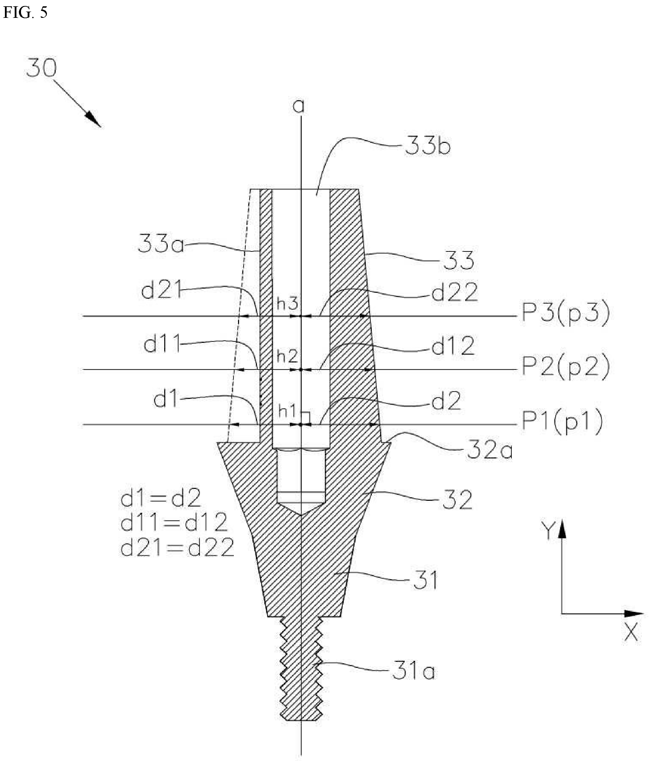

FIG. 4 is a schematic view for describing one example of a ready-made abutment, and FIG. 5 is a schematic cross-sectional view along direction "A-A" shown in FIG. 4.

A digital library including three-dimensional contour information of a ready-made abutment can be designed using a ready-made abutment 30 illustrated in FIGS. 4 and 5. For example, the ready-made abutment 30 may be an integrated screw type abutment in which a screw is integrally formed with a coupling portion 31. In addition, the ready-made abutment 30 is not limited to an integrated screw type abutment and may be a detachable screw type abutment in which a screw can be detached independently from the abutment.

The ready-made abutment 30 may be mainly divided into three portions although the structure and shape thereof may slightly vary depending on the manufacturer and states of an oral cavity and/or a treatment site of a patient. The ready-made abutment 30 includes the coupling portion 31 that is coupled to a fixture (not illustrated) placed in an alveolar bone, a cuff 32 that is formed on an upper portion of the coupling portion 31 and has a diameter that gradually increases upward, and a post 33 that is formed on an upper portion of the cuff 32 and supports a crown (not illustrated).

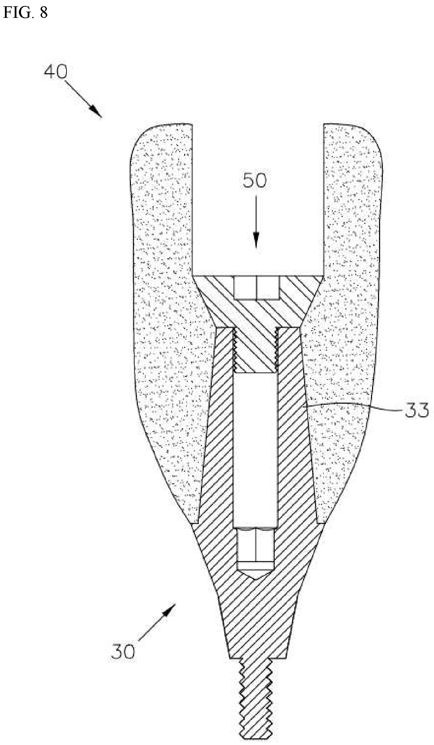

As in FIG. 5, in the ready-made abutment 30, a screw 31a is integrally formed with the coupling portion 31. The cuff 32 may be entirely or at least partially covered by gum in a state in which the coupling portion 31 is coupled and fixed to the fixture placed in the alveolar bone, and a berm 32a is provided at an upper surface of the cuff 32 (a boundary surface with the post 33). In a state in which a crown 40 (see FIG. 8) is coupled to the berm 32a to restore the post 33, a lower end of the crown 40 is seated on and supported by the berm 32a. The post 33 may have a tapered structure in which a diameter gradually decreases upward to facilitate coupling of the crown. For example, the post 33 may have a truncated cone shape as a whole, and a hollow 33b to which a fixing member 50 (see FIG. 8) is coupled may be formed in the center of the post 33.

In a method of designing a digital library according to an embodiment of the present invention, when designing three-dimensional contour information of the ready-made abutment 30 shown in FIGS. 4 and 5, the coupling portion 31 and the cuff 32 are designed to have almost the same contours (dimensions) as the coupling portion and the cuff of the actual ready-made abutment 30, and only the post 33 is designed using the proposed method. Therefore, design of a three-dimensional contour of the post 33 will be mainly described in this specification.

A cutting surface 33a and/or a cutting groove (see FIG. 2) are/is formed on an outer surface of the post 33 to prevent the crown from being rotated about the post 33 as an axis and distorted in a state in which the crown is coupled to restore the post 33. The number of cutting surfaces 33a and/or cutting grooves formed on the post 33 may be one or more than one. In addition, the cutting surface 33a and the cutting groove may be formed in an up-down direction (Y-axis direction) of the post 33 or may be formed in a left-right direction (X-axis direction) of the post 33. A cutting surface and a cutting groove may be formed together on the post 33.

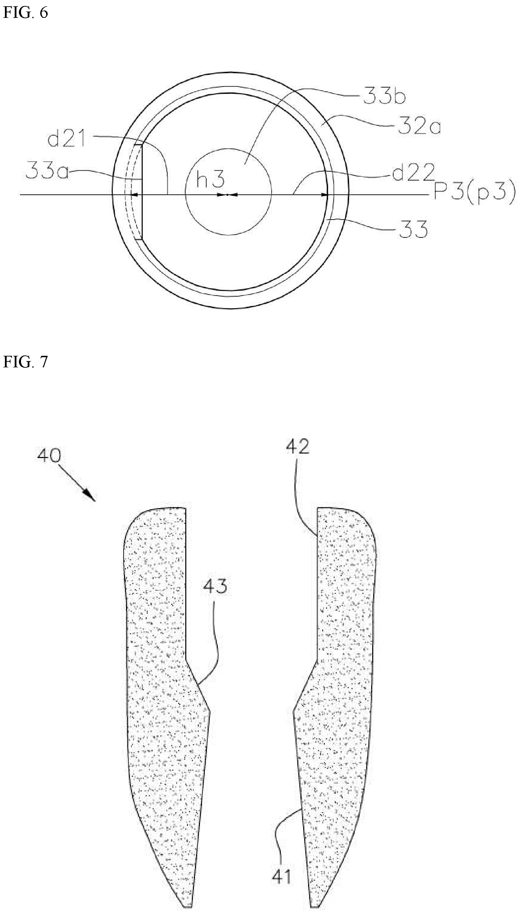

FIG. 6 is a schematic top view of the ready-made abutment illustrated in FIG. 4.

When a digital library of the ready-made abutment 30 illustrated in FIGS. 4 to 6 is designed using CAD or CAM, a three-dimensional contour is designed to exclude the cutting surface 33a and/or the cutting groove formed on the outer surface of the post 33, that is, to not reflect the cutting surface 33a and/or the cutting groove.

For example, when designing three-dimensional contour information of the ready-made abutment 30 having the structure shown in FIG. 4, the coupling portion 31 and the cuff 32 are designed to have the same contours as the coupling portion 31 and the cuff 32 of the ready-made abutment 30, and the post 33 is designed to have a three-dimensional contour formed in a truncated cone shape by ignoring the cutting surface 33a and/or the cutting groove formed on the outer surface of the post 33.

As in FIGS. 5 and 6, when lines p1, p2, and p3 that perpendicularly intersect an axial line a, which is a central axis of the post 33, on arbitrary planes P1, P2, and P3 are referred to as perpendicular lines p1, p2, and p3 relative to the axial line a, and points at which the axial line a intersects the perpendicular lines p1, p2, and p3 on the arbitrary planes P1, P2, and P3 are referred to as intersection points h1, h2, and h3, when designing the three-dimensional contour of the post 33, the three-dimensional contour of the post 33 is designed so that a distance between each of the intersection points h1, h2, and h3 and the outer periphery (circumferential surface) of the post 33 has the same length on the same plane P1, P2, or P3 along the circumference of the post 33. In this way, even when the cutting surface 33a and/or the cutting groove are/is formed on the post 33 of the ready-made abutment 30, the cutting surface 33a and/or the cutting groove may be ignored when designing the digital library.

For example, when designing the three-dimensional contour of the post 33 of the ready-made abutment 30, as in FIGS. 5 and 6, the three-dimensional contour of the post 33 is designed so that distances d1 and d2 between the intersection point h1 and the outer periphery of the post 33 are the same as each other on the plane P1 along the circumference of the post 33, distances d11 and d12 between the intersection point h2 and the outer periphery of the post 33 are the same as each other on the plane P2 along the circumference of the post 33, and distances d21 and d22 between the intersection point h3 and the outer periphery of the post 33 are the same as each other on the plane P3 along the circumference of the post 33. That is, based on each intersection point at which the axial line and each perpendicular line intersect, a distance between each intersection point and the outer periphery of the post 33 may be formed to be the same on the same plane along the circumference of the post 33.

When a distance between the corresponding intersection point and the outer periphery of the post 33 is designed to be the same on the same plane along the circumference of the post 33, a distance between a portion of the outer periphery of the post 33 where the cutting surface 33a and/or the cutting groove is formed and the corresponding intersection point is designed to match a distance between a portion of the outer periphery of the post 33 where the cutting surface 33a and/or the cutting groove is not formed and the corresponding intersection point. For example, when designing the three-dimensional contour of the post 33, the three-dimensional contour of the post 33 is designed so that the distance d21 between the intersection point h3 and the outer periphery of the post 33 where the cutting surface 33a is formed (the left side in FIG. 6) matches the distance d22 between the intersection point h3 and the outer periphery of the post 33 where the cutting surface 33a is not formed (the right side in FIG. 6) on the plane P3.

In this way, when designing a digital library in an embodiment of the present invention, a three-dimensional contour related to the contour of the post 33 is designed so that a distance between a portion of the outer periphery of the post 33 where the cutting surface 33a and/or the cutting groove is formed and the intersection point matches a distance between a portion of the outer periphery of the post 33 where the cutting surface 33a and/or the cutting groove is not formed and the corresponding intersection point on the same plane. Here, the distance between the portion of the outer periphery of the post 33 where the cutting surface 33a and/or the cutting groove is not formed and the intersection point may be the same as a diameter of the post 33 on the corresponding plane. In this way, even when the cutting surface 33a and/or the cutting groove are/is formed on the post 33 of the ready-made abutment 30, it is possible to design a digital library that includes three-dimensional contour information of the ready-made abutment that does not reflect the cutting surface 33a and/or the cutting groove. That is, it is possible to design three-dimensional contour information of the post having a truncated cone shape as a whole.

FIG. 7 is a view schematically illustrating a cross-section of a crown according to one example of the present invention.

The crown 40 having the structure shown in FIG. 7 is designed using the digital library designed above. The three-dimensional contour information of the abutment 30, in particular, contour information of the post 33, included in the digital library determines an inner shape of an accommodating portion 41 of the crown 40.

The accommodating portion 41 of the crown 40 is a portion into which the post 33 of the ready-made abutment 30 is inserted and to which the post 33 is closely shape-matched, and the accommodating portion 41 of the crown 40 corresponds to the three-dimensional contour of the abutment because the inner shape of the accommodating portion 41 of the crown 40 is determined based on the digital library designed using the method described above. Accordingly, when the manufactured crown 40 is coupled and fixed to the post 33 of the ready-made abutment 30, the outer periphery of the post 33 may be uniformly coupled to an inner surface of the accommodating portion 41 of the crown 40.

Currently, most ready-made abutments have a post on which a cutting surface and/or a cutting groove are/is formed. Accordingly, when a digital library is designed by scanning or using CAD or CAM, a three-dimensional contour of an abutment is designed to reflect the cutting surface and/or the cutting groove of the post. When a crown is manufactured using the digital library designed in the above state in which the cutting surface and/or the cutting groove are/is reflected, early contact occurs between the manufactured crown and the post. The early contact problem may deepen when an error occurs in manufacturing the crown.

Therefore, in a method of manufacturing a crown according to an embodiment of the present invention, when designing a digital library for manufacturing a crown to be coupled to a ready-made abutment having a post on which a cutting surface and/or a cutting groove are/is formed, a three-dimensional contour of the post is designed in a truncated cone shape based on an outer periphery reflecting the overall contour of the post while ignoring the cutting surface and/or the cutting groove. Therefore, even when an error occurs in the process of manufacturing the crown in a dental laboratory, the occurrence of early contact between the manufactured crown and the post can be fundamentally prevented.

As in FIGS. 4 and 5, the ready-made abutment 30 may be formed of a tapered structure in which a diameter of the post 33 gradually decreases upward or may be formed of a structure in which a diameter of the post 33 does not change. In addition, the ready-made abutment may be coupled and fixed to a fixture placed in an alveolar bone through the integrated screw type structure (the structure in which the screw is integrally formed with the coupling portion) or the detachable screw type structure (the structure in which the screw is inserted into the hollow 33b formed in the center of the abutment and is coupled and fixed to the fixture).

FIG. 8 is a view schematically illustrating a cross-section of an implant system including a crown manufactured through an embodiment of the present invention and is a cross-sectional view along direction "B-B" shown in FIG. 4.

Referring to FIGS. 7 and 8, an implant system includes the abutment 30 and the crown 40 (for example, a single crown) that restores the post 33 of the abutment 30. A hole 42 into which the fixing member 50 for coupling and fixing the crown 40 to the abutment 30 is inserted is formed in an upper portion of the crown 40. The hole 42 is buried later. A support step portion 43 at which a head portion of the fixing member 50 is supported downward is formed between the hole 42 and the accommodating portion 41. The fixing member 50 may have a screw portion and may be screw-coupled to the hollow 33b of the post 33.

In a method of designing a digital library according to an embodiment of the present invention, the digital library is designed based on the three-dimensional contour of the post 33 of the ready-made abutment 30, and the crown 40 is manufactured using the digital library designed in this way. Accordingly, the manufactured crown 40 does not reflect the cutting surface 33a and/or the cutting groove formed on the post 33. Preferably, the shape of the inner surface of the accommodating portion 41 of the manufactured crown 40 does not reflect the cutting surface 33a and/or the cutting groove formed on the post 33. That is, the inner surface of the accommodating portion 41 of the crown 40 may be formed in a truncated cone shape to correspond to the contour of the post 33. Therefore, when the manufactured crown 40 is coupled to the abutment 30 in a state in which the cutting surface 33a and/or the cutting groove formed on the post 33 are/is not reflected, the inner surface of the accommodating portion 41 of the crown 40 may be uniformly coupled to the outer periphery of the post 33 without the occurrence of early contact that has been pointed out as a problem of the conventional method.

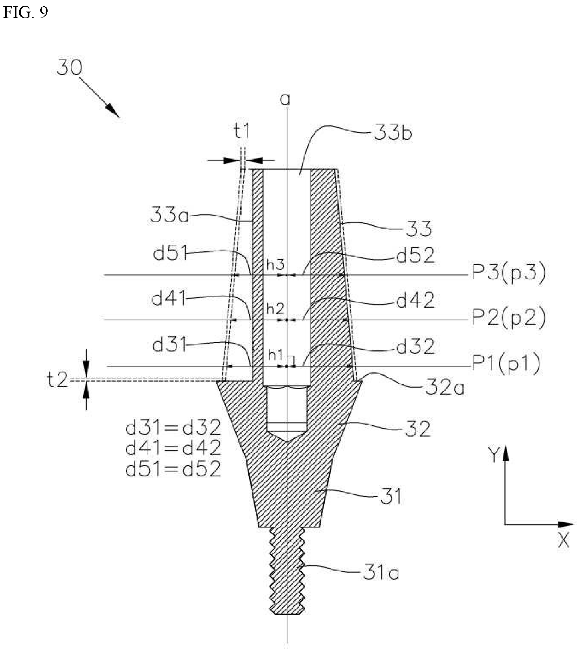

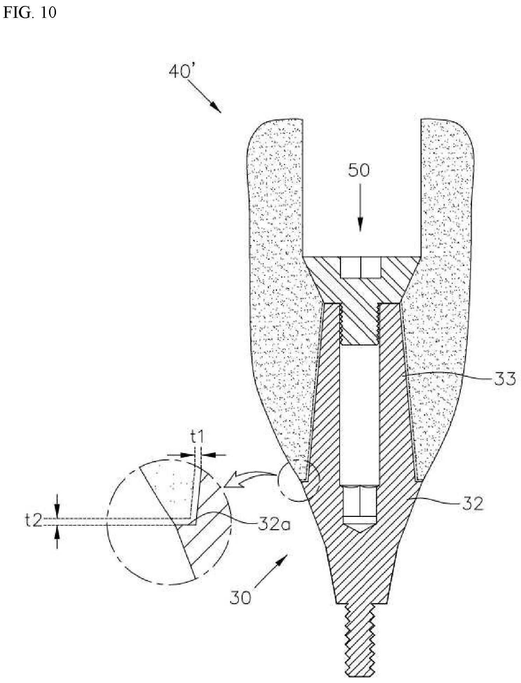

FIG. 9 is a view for describing a method of designing a digital library according to another example of the present invention and is a schematic cross-sectional view of a ready-made abutment along direction "A-A." FIG. 10 is a schematic cross-sectional view along direction "B-B" of an implant system including a crown manufactured through the method of FIG. 9.

Referring to FIGS. 9 and 10, a method of designing a digital library according to another example of the present invention is provided as a digital library designing method that is applied to an implant system in which an arbitrary member (not illustrated) is interposed between a crown 40' and an abutment 30 for a predetermined purpose.

As in FIG. 10, in an implant system, a cushioning member (an elastic body or the like) is interposed between the crown 40' and the abutment 30 to cushion an occlusion force applied to the crown 40' during mastication. Here, the cushioning member may extend to between an outer periphery of the abutment 30 and an inner surface of an accommodating portion of the crown 40' and between a berm 32a and a lower end of the abutment 30.

Therefore, in the method of designing a digital library according to another example of the present invention, when designing a three-dimensional contour of the ready-made abutment 30, the three-dimensional contour is designed in consideration of the thickness and volume of the cushioning member. For example, as in FIG. 9, when designing the three-dimensional contour of the ready-made abutment 30, the contour is designed to be larger by as much as a thickness t1 at an outer periphery of a post 33 and by as much as a thickness t2 at the berm 32a. Here, the thicknesses t1 and t2 may each be equal to or slightly smaller than the thickness of the cushioning member. Preferably, the thicknesses t1 and t2 are each formed to be slightly smaller than the thickness of the cushioning member by taking into consideration the degree to which the cushioning member is elastically deformed by a pressing force applied to the cushioning member in a state in which the crown 40' is coupled. In addition, the thicknesses t1 and t2 may be equal to or different from each other.

When the crown 40' is manufactured using the digital library designed using the method described above with reference to FIG. 9, as in FIG. 10, an interval (a gap) that corresponds to the thickness t1 is formed between an inner surface of an accommodating portion of the crown 40' and the outer periphery of the post 33, and an interval that corresponds to the thickness t2 is formed between the berm 32a and a lower end of the crown 40'. In this way, in the method of designing a digital library according to another example of the present invention, the digital library is designed so that, in consideration of the thickness or volume of the cushioning member interposed between the crown and the abutment, an inner portion of the accommodating portion of the crown has an accommodating space that is larger than the overall contour of the post. That is, when designing the digital library, the digital library is designed to reflect the thickness and the volume of the cushioning member in advance so that a space into which the cushioning member will be inserted is secured, and thus the cushioning material can be more stably inserted and installed between the crown and the abutment.

While preferred embodiments of the present invention have been described above using specific terms and illustrated, such terms are merely for clearly describing the present invention. In addition, it is self-evident that changes and variations may be made to the embodiments of the present invention and the used terms without departing from the technical spirit or scope of the following claims. Modified embodiments should not be understood as being separate from the spirit or scope of the present invention and should be considered as falling within the scope of the claims of the present invention.

### [Description of reference numerals]

10, 20, 30: abutment
11, 21, 33: post
11a, 33a, cutting surface
21 a: cutting groove
31: coupling portion
31a: screw
32: cuff
32a: berm
33b: hollow
40, 40': crown
41: accommodating portion
42: hole
43: support step portion
50: fixing member

## Claims

1. A method of manufacturing a crown of a dental implant using a digital library, the method comprising:
a process of designing a digital library including three-dimensional contour information of a ready-made abutment including a post having at least one of a cutting surface and a cutting groove formed on an outer periphery thereof; and
a process of manufacturing a crown using the designed digital library,
wherein, when designing the three-dimensional contour information of the ready-made abutment in the process of designing the digital library, the three-dimensional contour information of the ready-made abutment is designed so that, based on an intersection point at which an axial line of the post and a line perpendicular to the axial line of the post intersect, a distance between the intersection point and the outer periphery of the post is the same on the same plane along the circumference of the post.

2. A method of manufacturing a crown of a dental implant using a digital library, the method comprising:
a process of designing a digital library including three-dimensional contour information of a ready-made abutment including a post having at least one of a cutting surface and a cutting groove formed on an outer periphery thereof; and
a process of manufacturing a crown using the designed digital library,
wherein, when designing the three-dimensional contour information of the ready-made abutment in the process of designing the digital library, a three-dimensional contour of the post is designed in consideration of a thickness or a volume of a member interposed between the crown and the ready-made abutment.

3. A method of manufacturing a crown of a dental implant using a digital library, the method comprising:
a process of designing a digital library including three-dimensional contour information of a ready-made abutment; and
a process of manufacturing a crown using the designed digital library,
wherein, when designing the three-dimensional contour information of the ready-made abutment in the process of designing the digital library, a three-dimensional contour of the post is designed in consideration of a thickness or a volume of a member interposed between the crown and the ready-made abutment.

4. The method of any one of claims 1 to 3, wherein, when designing the three-dimensional contour information of the ready-made abutment in the process of designing the digital library, the three-dimensional contour of the post is designed in a truncated cone shape.

5. The method of claim 2, wherein the member is an elastic body.

6. The method of any one of claims 1 to 3, wherein, when designing the three-dimensional contour information of the ready-made abutment in the process of designing the digital library, dental computer aided design (CAD) or dental computer aided manufacturing (CAM) is used.

7. The method of claim 1, wherein, when designing the three-dimensional contour information of the ready-made abutment so that the distance between the intersection point and the outer periphery of the post is the same on the same plane along the circumference of the post in the process of designing the digital library, the three-dimensional contour information of the ready-made abutment is designed so that a distance between the intersection point and a portion of the outer periphery of the post where the cutting surface or the cutting groove is formed matches a distance between the intersection point and a portion of the outer periphery of the post where the cutting surface or the cutting groove is not formed.

8. The method of any one of claims 1 to 3, wherein the post is formed of a tapered structure in which a diameter gradually decreases upward.

9. The method of any one of claims 1 to 3, wherein the ready-made abutment is formed of an integrated screw type structure or a detachable screw type structure.

10. The method of any one of claims 1 to 3, wherein the crown is a single crown or a bridge crown.
